# EUROPEAN PATENT APPLICATION

(11) **EP 1 358 885 A1**
(43) Date of publication of application: **05.11.2003**
(21) Application number: 02729382.8
(22) Date of filing: 10.01.2002
(51) Int. Cl.: A61K 31/765, A61P 37/08, A61P 11/02, A23L 1/30, C08G 63/06

(54) **ANTIALLERGIC AGENTS**

(30) Priority: 12.01.2001 JP 2001004823
(71) Applicant: AMATO PHARMACEUTICAL PRODUCTS, LTD., Fukuchiyama-shi, Kyoto 620-0932 (JP)
(72) Inventor: MURAKAMI, Masahiro, Osaka-shi, Osaka 547-0026 (JP); KOHNO, Shigekatsu, Ibi-gun, Gifu 503-2406 (JP); NABE, Takeshi, Chuo-ku, Osaka-shi, Osaka 540-0024 (JP)
(74) Representative: Sternagel, Fleischer, Godemeyer & Partner Patentanwälte
(86) International application number: JP0200079
(87) International publication number: WO02055091

(57) **Abstract**

The object of the present invention is to provide a novel antiallergic agent which can be used for therapy and prevention of allergic diseases such as allergic rhinitis. According to the present invention, there is provided an antiallergic agent which comprises a mixture of cyclic and/or straight chain poly lactic acids having a condensation degree of 3 to 20.

## Description

### TECHNICAL FIELD

The present invention relates to an antiallergic agent. More specifically, the present invention relates to an antiallergic agent which can be used as a medicament, food for special healty uses, health food and the like which are effective for allergic diseases such as allergic rhinitis.

### BACKGROUND ART

An allergic reaction is classified into 4 types of type I to type IV on the basis of differences in mechanisms. Among them, the type I allergic reaction normally causes allergic diseases such as allergic rhinitis, bronchial asthma and hives. The type I allergic reaction, also named immediate allergy, is a biological reaction in which an allergen-specific IgE antibody is bound to a receptor on a surface of a mast cell in tissue and a blood basophil and then an allergen is bound to the IgE antibody to discharge an excess of a chemical mediator such as histamine and leukotriene from the mast cell and the basophil, thereby causing various allergic reactions. Therefore, for the therapy of a type I allergic disease, an antihistamine agent and an antiallergic agent that has an action of suppressing the release of the chemical mediator from the mast cell, are used. However, such antihistamine agent and antiallergic agent frequently shows an adverse effect, and hence a problem of safety arises in repeated uses over a long term.

Diseases such as allergic contact dermatitis are caused by the type IV allergic reaction ordinarily called delayed allergy. A steroid agent is used for the therapy of the type IV allergic diseases. The steroid agent is particularly effective for the suppression of cytokine production and the treatment of eczema. However, in the case of large amount or long term use, severe adverse effect highly probably occurs and a problem of safety remains.

Among the above described allergic diseases, allergic rhinitis is a representative type I allergic disease having three major features, sneeze, rhinorrhea secretion acceleration, and nasal obstruction. It is suggested that histamine released by an antigen-antibody reaction is largely involved in sneeze and rhinorrhea secretion sthenia. On the contrary, it is presumed that occurrence of nasal obstruction is attributable to an edema caused by sthenia of vasodilation and blood vessel permeability. However, its detailed mechanism has not been elucidated.

The present inventors have previously established a model animal of allergic rhinitis which is considered to be similar with human clinical condition, by transnasally sensitizing a Guinea pig by administering the antigen which was extracted from cedar pollens and adsorbed to Al(OH)₃, to its nasal cavity and then subjecting the Guinea pig to the repeated inhalation of cedar pollen particles. In this model animal, not only an expression of sneeze was observed mainly within 1 hour after the reaction occurred, but also an increase of specific airway resistance (sRaw), which is an index of nasal obstruction, was observed as two phases with two peaks at 1 and 4 hours after the reaction occurred. In addition, in this model animal, at 4 hours to 2 days after the reaction occurred, sthenia of nasal anaphylaxis which prominently reacts to nose drop of histamine and leukotriene D₄, is observed.

From studies that have been made so far, it has been reported that a mixture of cyclic and/or straight chain poly L-lactic acids having a condensation degree of 3 to 20 is useful as an antineoplastic agent (Japanese Patent Application Laying-Open (Kokai) Nos. 9-227388 and 10-130153). However, evaluation of antiallergic effect of a mixture of cyclic and/or straight chain poly L-lactic acids having a condensation degree of 3 to 20 has not been reported.

### DISCLOSURE OF THE INVENTION

An object of the present invention is to provide a novel antiallergic agent which can be used for therapy and prevention of allergic diseases such as allergic rhinitis. Further, another object of the present invention is to provide antiallergic food and drink using the aforementioned antiallergic agent.

In order to study for the purpose of solving the aforementioned objects, the present inventors have administered a mixture of cyclic and/or straight chain poly lactic acids having a condensation degree of 3 to 20 to a model animal of allergic rhinitis, and have examined an effect of the mixture of poly lactic acids on the sneeze occurred after the antigen-antibody reaction, the two phase nasal obstruction, and the expression of nasal anaphylaxis against histamine. As a result, it has been found that the mixture of poly lactic acids used in the present invention suppresses two phase nasal obstruction and also suppresses the expression of nasal anaphylaxis against histamine. The invention was completed on the basis of these findings.

Thus, according to the present invention, there is provided an antiallergic agent which comprises a mixture of cyclic and/or straight chain poly lactic acids having a condensation degree of 3 to 20.

The antiallergic agent according to the present invention can be used as a therapeutic agent or a preventive agent of allergic diseases. Examples of the allergic diseases include the type I allergy disease such as allergic rhinitis.

Preferably, the lactic acid that is a repeating unit in the poly lactic acid consists substantially of L-lactic acid.

Preferably, the mixture of cyclic and/or straight chain poly lactic acids having a condensation degree of 3 to 20 is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

Preferably, condensation by dehydration is performed by stepwise decompression and temperature rise under nitrogen gas atmosphere.

Preferably, reverse phase column chromatography is performed by ODS column chromatography.

According to another aspect of the present invention, there are provided antiallergic food and drink which comprises the aforementioned antiallergic agent of the present invention.

According to still another aspect of the present invention, there is provided the use of a mixture of cyclic and/or straight chain poly lactic acids having a condensation degree of 3 to 20 in the production of an antiallergic agent or antiallergic food and drink.

According to a still further aspect of the present invention, there is provided a method for suppressing allergy, which comprises a step of administering an effective amount of a mixture of cyclic and/or straight chain poly lactic acids having a condensation degree of 3 to 20 to mammals such as humans.

### BRIEF DESCRIPTION OF THE DRAWINGS

Figure 1 shows a mass spectrum of the mixture of poly lactic acids obtained by Production Example 1.

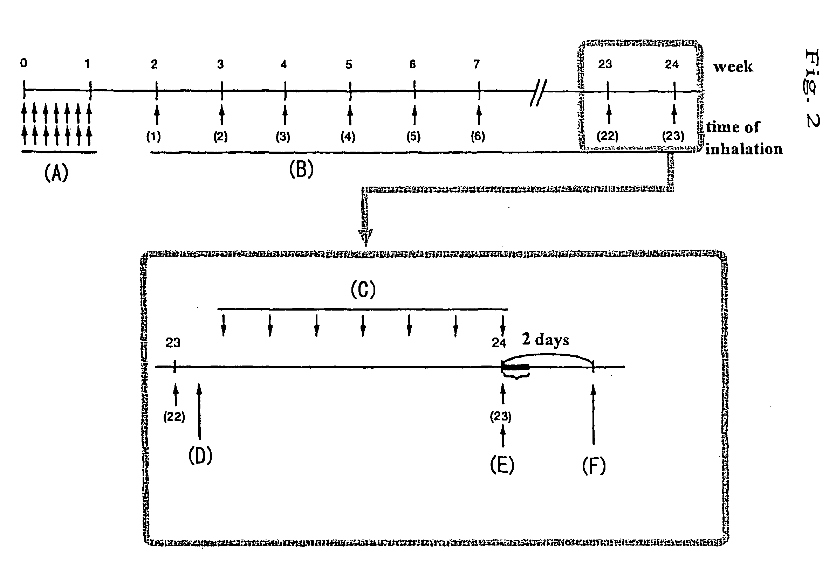

Fig. 2 shows a flow chart of experimental procedures of Test Example 1. In Fig. 2, (A) shows sensitization by inhalation of the cedar pollen extract in the nasal cavity (0.6 µg protein/0.6 mg Al(OH)₃/animal). (B) shows administration/sensitization by inhalation of cedar pollens (about 3.6 mg/animal/dose). (C) shows oral administration of CPL (100 mg/2 ml/kg) or a vehicle (2 ml/kg). (D) shows division into 2 groups (A and B). (E) shows an evaluation of the effect of CPL on the two phase nasal cavity resistance and sneeze induced by administration of the antigen. (F) shows the evaluation of the effect of CPL on the expression of nasal anaphylaxis against histamine.
Fig. 3 shows the change with time of the specific airway resistance (sRaw) induced by the 22nd inhaling administration of cedar pollens to the sensitized Guinea pig.
Fig. 4 shows the effect of CPL on the two phase increase of the specific airway resistance (sRaw) induced by the 23rd inhaling administration of cedar pollens to the sensitized Guinea pig.
Fig. 5 shows the effect of CPL on the sneeze induced by the 23rd inhaling administration of cedar pollens to the sensitized Guinea pig.
Fig. 6 shows the effect of CPL on the expression of nasal anaphylaxis against histamine in the sensitized Guinea pig.

### THE BEST MODE FOR CARRYING OUT THE INVENTION

The embodiment and method for the practice of the present invention are described in detail below.

The antiallergic gent of the present invention comprises, as an active ingredient, a mixture of cyclic and/or straight chain poly lactic acids having a condensation degree of 3 to 20, and can be used as a therapeutic agent or a preventive agent of allergic diseases such as allergic rhinitis.

Allergic diseases are diseases caused by a tissue injury based on the reaction of an exogenous antigen with a specific antibody thereto or a sensitized lymphocyte. The allergic reactions are classified into 4 types of type I, type II, type III and type IV according to its reaction modes. Type I allergy is a reaction with an IgE antibody being involved, and is named immediate type, IgE-dependent type or anaphylaxis type. Type II allergy is also called cell injury type or cytolysis type, and is involved in the onset of diseases such as incompatible blood transfusion, autoimmune hemolytic anemia and sudden thrombocytopenia. When IgG or IgM antibody which was produced against the exogenous antigen or an auto-antigen binds to a target cell, a complement system is activated, and the target cells are injured. Type III allergy is called immune complex type or Arthus type, and their major diseases are serum disease, glomerulonephritis, systemic erythematodes, hypersensitivity pneumonitis, and the like. This allergy is a tissue injury which is caused by an event where IgG antibody or IgM antibody in blood is bound to a soluble antigen to form an immune complex. Type IV allergy is also called delayed type or cellular immunity type. As observed in the tuberculin reaction, an inflammation reaction characterized by erythema and induration is present at 48 to 72 hours after injection of the antigen.

In the type I allergy, house dust, mites, pollens, fungi, animal hair dirt or the like in a living body before onset of a disease irrupt into the living body as an antigen via an airway, a digestive tract, a skin and the like, resulting in the production of an IgE antibody. The IgE antibody binds to a high affinity IgE receptor on the mast cell and the basophil to sensitize these cells. Thereafter, the IgE antibody is exposed to the antigen and the antigen is bound to the IgE antibody. Thus, cross-linking of the high affinity IgE receptor and activation of the cell occur. Then, degranulation and production and release of the chemical mediator from a cell membrane lipid occur. Histamine, eosinophil chemotactic factor, neutrophil chemotactic factor and the like are released from the granules, and as a new chemical mediator, prostaglandins, thromboxane, leuokotriene, platelet activating factor and the like are produced. Inflammation cells move and penetrate into a lesion by these chemical mediators and cytokines.

As the diseases caused by the type I allergy, there are many common diseases such as atopic dermatitis, bronchial asthma, pollinosis, hives and allergic rhinitis. The antiallergic agent of the present invention can be widely used for therapy and prevention of allergic diseases, and particularly can be used for therapy and prevention of diseases caused by the type I allergy reaction.

The antiallergic agent of the present invention or the food and drink comprising the same can be used not only for therapy of an allergic symptom, but also for prevention of occurrence of the allergic symptom and/or the preventive therapy to relieve the symptom.

In the antiallergic agent and the antiallergic food and drink of the present invention, a mixture of cyclic and/or straight chain poly lactic acids having a condensation degree of 3 to 20 is used as an active ingredient.

The term "a mixture of poly lactic acids" used in the present invention means a mixture wherein cyclic and/or straight chain poly lactic acids having a condensation degree of 3 to 20 are present at any ratio. That is to say, the term "mixture" does not only mean a mixture of poly lactic acids having any condensation degree ranging from 3 to 20, but is also used as a concept including a mixture of cyclic and straight chain poly lactic acids. As is described below in the present specification, "a mixture of poly lactic acids" can be obtained by condensing lactic acids by dehydration and then performing purification by a suitable method. Although the term "a mixture of poly lactic acids" is used in the present specification for the sake of convenience, this term also includes a poly lactic acid consisting of a single ingredient such as a cyclic poly lactic acid having single condensation degree or a straight chain poly lactic acid having single condensation degree.

The term "condensation degree" is used to mean the number of lactic acid unit that is a repeating unit in poly lactic acids. For example, the cyclic poly lactic acid is assumed to have the following structural formula wherein n represents condensation degree (n = 3 to 20).

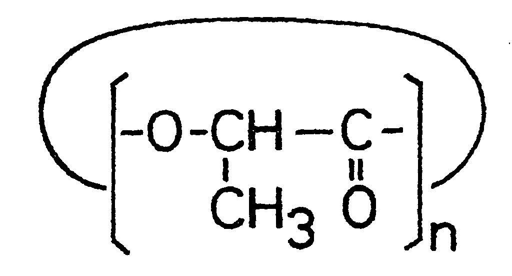

When "lactic acid" is simply referred to in the present specification, this lactic acid includes all of L-lactic acid, D-lactic acid or a mixture comprising these types of lactic acid at any ratio. Preferably in the present invention, the lactic acid consists substantially of L-lactic acid. The term "substantially" is used herein to mean that the ratio of L-lactic acid units in a mixture of poly lactic acids (number of L-lactic acid unit / number of L-lactic acid unit + number of D-lactic acid unit × 100) is, for example, 70% or more, preferably 80% or more, more preferably 85% or more, further more preferably 90% or more, and particularly preferably 95% or more. The ratio of L-lactic acid units in a mixture of poly lactic acids depends on the ratio of L-lactic acid and D-lactic acid that exist in lactic acids used as a starting substance.

The methods for producing a mixture of cyclic and/or straight chain poly lactic acids having a condensation degree of 3 to 20 are not particularly limited, and the mixture of poly lactic acids can be obtained by the production methods described, for example, in Japanese Patent Application Laying-Open (Kokai) Nos. 9-227388 and 10-130153 or Japanese Patent Application No. 11-39894 (All publications cited herein are incorporated herein by reference in their entirety).

More specifically, for example, a mixture of cyclic and/or straight chain poly lactic acids having a condensation degree of 3 to 20 can be obtained by the following method A.

### Method A:

First, lactic acid (preferably, lactic acid substantially consisting of L-lactic acid) is condensed by dehydration under an inactive atmosphere. Examples of the inactive atmosphere include nitrogen gas and argon gas, and nitrogen gas is preferred.

Dehydration and condensation reaction is carried out at a temperature of 110°C to 210°C, preferably 130°C to 190°C under normal pressure to reduced pressure of approximately 1mmHg, and particularly preferably the reaction is carried out by stepwise decompression and stepwise temperature rise. A reaction period can be determined as appropriate. For example, the reaction can be carried out for 1 to 20 hours. Where stepwise decompression and stepwise temperature rise are applied, reaction is performed by dividing the reaction period into two or more partial reaction periods, and then determining pressure and temperature for each of the reaction periods. Where stepwise decompression is applied, pressure can be reduced, for example, from a normal pressure to 150mmHg and then to 3mmHg. Where stepwise temperature rise is applied, temperature can be raised, for example, from 145°C to 155°C and then to 185°C. Practically, the reaction can be carried out by using these conditions in combination, for example, 145°C, normal pressure, 3 hours; 145°C, 150mmHg, 3 hours; 155°C, 3mmHg, 3 hours; and 185°C, 3mmHg, 1.5 hours.

Subsequently, ethanol and methanol are added to the reaction mixture obtained by the dehydration and condensation reaction, and the mixture is filtered. The obtained filtrate is dried to obtain ethanol- and methanol-soluble fractions. The term "ethanol- and methanol-soluble fractions" is used in the present specification to mean fractions soluble in a mixed solution of ethanol and methanol. In order to obtain ethanol and methanol-soluble fractions, a reaction mixture obtained by dehydration and condensation reaction is mixed with ethanol and methanol, where the ratio of ethanol and methanol can be determined as appropriate. For example, the ratio is ethanol:methanol = 1 : 9. The order, method and the like for adding ethanol and methanol to a reaction mixture are not limited, and may be selected as appropriate. For example, ethanol may be added at first to the reaction mixture obtained by the dehydration and condensation reaction, and then methanol may be added thereto.

The thus obtained ethanol- and methanol-soluble fractions are subjected to reverse phase column chromatography, especially to chromatography where an octadecylsilane (ODS) column is used. First, fractions eluted with 25 to 50 weight % acetonitrile aqueous solution of pH 2 to 3 are removed, and then fractions eluted with 90 weight % or more acetonitrile aqueous solution of pH 2 to 3, preferably 99 weight % or more acetonitrile aqueous solution, are collected so as to obtain a mixture of cyclic and/or straight chain poly lactic acids having a condensation degree of 3 to 20.

The thus obtained mixture of cyclic and/or straight chain poly lactic acids is neutralized with an alkaline substance such as sodium hydroxide, and is dried under reduced pressure, and then according to standard techniques, the mixture can be formulated in a desired form as mentioned below.

Other examples of the methods for producing a mixture of cyclic and/or straight chain poly lactic acids having a condensation degree of 3 to 20 used in the present invention include a method described in Japanese Patent Application No. 11-265715 (hereinafter referred to as method B), or a method described in Japanese Patent Application No. 11-265732 (hereinafter referred to as method C) (All publications cited herein are incorporated herein by reference in their entirety). Methods B and C will be described specifically below.

### Method B:

Method B is a method for producing a cyclic lactic acid oligomer which comprises polymerizing lactid in the presence of a lithium compound represented by RYLi [wherein R represents an aliphatic group or aromatic group, Y represents oxygen atom or sulfur atom]. In the case of performing the polymerization reaction, the ratio of the amounts of the lithium compound (RYLi) is 1-0.1 mol, preferably 0.2-0.3 mol per mol of lactide. The reaction temperature is -100 to 0°C, preferably -78 to -50°C. Reaction is preferably carried out by starting from a temperature of -78 to -50°C and gradually raising it to room temperature. The reaction is preferably carried out in the presence of a reaction solvent. As the reaction solvent, there can be used, for example, a cyclic ether such as tetrahydrofuran, diethylether, and dimethoxyethane. The reaction atmosphere can be an inactive gas atmosphere such as nitrogen gas and argon. The reaction pressure is not limited, and is preferably a normal pressure.

The composition (that is, the mixing ratio of cyclic lactic acid oligomer and a chain lactic acid oligomer) of the lactic acid oligomer obtained as described above fluctuates depending on the lithium compound used as a reaction assistant. Where a lithium compound of alkyl alcohol having a carbon number of 1 to 3 (ROLi) (wherein R represents an alkyl group with carbon number 1 to 3) is used as a lithium compound, a mixture of a cyclic lactic acid oligomer and a chain oligomer (proportion of the cyclic lactic acid oligomer: 80 to 85 weight %) is obtained. When a lithium compound of alkyl alcohol having a carbon number of 4 or more such as t-butyl alcohol, or thiophenol compound is used as a lithium compound, substantially only a cyclic lactic acid oligomer can be selectively obtained.

### Method C:

This method comprises:
(i) a first heating step which comprises heating lactic acid under a pressure condition of 350 to 400 mmHg and to a temperature of 120 to 140°C so as to perform dehydration and condensation, and distilling off and removing only by-product water without distilling lactid off;
(ii) a second heating step for synthesizing a product condensed by dehydration comprising chain lactic acid oligomers as the main ingredient, which comprises, after completion of the first heating step, heating the reaction product to a temperature of 150 to 160°C while reducing the reaction pressure to 15 to 20 mmHg at a decompression rate of 0.5 to 1 mmHg/min, wherein only by-product water is distilled off and removed while avoiding distillation of lactid; and after the reaction pressure is reduced to 15 to 20 mmHg, maintaining the reaction under the same pressure condition and at a reaction temperature of 150 to 160°C;
(iii) a third heating step for synthesizing cyclic oligomers which comprises, after completion of the second heating step, heating under a pressure condition of 0.1 to 3 mmHg and at 150 to 160°C to cyclize the chain lactic oligomer.

In this method, first, in the first heating step, lactic acid is heated under reduced pressure to perform dehydration and compression reaction. In this case the reaction period is 3 to 12 hours, preferably 5 to 6 hours. To allow the reaction in the first heating step to proceed smoothly, by-product water produced by condensation of lactic acids by dehydration is distilled off. At this time, distillation of by-product water is performed such that lactid, which is the dehydrated condensed product of two molecules of lactic acid, is not distilled off. To achieve such purpose, the reaction pressure is maintained at a reduced pressure, preferably 300 to 500 mmHg, more preferably 350 to 400 mmHg. Under this pressure condition, heating is performed at a temperature range of 100 to 140°C, preferably 130 to 140°C. The reaction product produced by reaction in the first heating step mainly comprises as the main ingredient a dehydrated condensed product of 3 to 23 molecules of lactic acid.

To obtain oligomers having an increased average degree of polymerization in the second heating step after completion of the above first heating step, heating is performed at a temperature higher than the reaction temperature of the above first heating step, preferably at 145°C to 180°C, more preferably 150°C to 160°C, while the reaction pressure is reduced to 10 to 50 mmHg, preferably 15 to 20 mmHg, so that dehydration and condensation reaction is further continued.

As with the reaction in the above first heating step, reaction is performed under a condition where by-product water, but not lactid, is distilled off, to allow the reaction to proceed smoothly. The rate at which reaction pressure is reduced to a pressure in the above range (decompression rate) is normally required to be maintained within a range of 0.25 to 5 mmHg/min, preferably 0.5 to 1 mmHg/min, in order to avoid distillation of lactid and increase the reaction efficiency. A decompression rate lower than the above range is not preferred because it will increase the time required to reduce pressure to a given pressure. On the other hand, a decompression rate higher than the above range is also not preferred because it will cause lactid to be distilled off together with by-product water.

After the reaction pressure is reduced to a certain pressure, reaction is further continued at that reaction pressure. The heating time period in this case is 3 to 12 hours, preferably 5 to 6 hours.

A lactic acid oligomer having an average polymerization degree of 3 to 30, preferably 3 to 23 is obtained by the reaction in the above second heating step. The proportion of cyclic oligomers in the oligomers in this case is normally about 70 to 80 weight %.

In the third heating step, after completion of the above second heating step, a reaction pressure is maintained at 0.25 to 5 mmHg, preferably 0.5 to 1 mmHg, and reaction is further continued at a temperature of 145 to 180°C, preferably 150 to 160°C. A reaction period is 3 to 12 hours, preferably 5 to 6 hours. By-product water produced in this case is also distilled off. In this case, distillation of lactid is preferably avoided. However, since the reaction product contains almost no lactid, it is not required to specially lower the decompression rate.

Lactic acid oligomers produced by reaction in the above third heating step have an average polymerization degree of 3 to 30, preferably 3 to 23, and contain cyclic oligomer in the proportion of 90 weight % or more, preferably 99 weight % or more.

The above methods A, B and C merely show some of specific examples of methods of producing a mixture of poly lactic acids used in the present invention. A mixture of poly lactic acids which is produced by other methods can also be used in the present invention.

The antiallergic agent of the present invention can be prepared by optionally selecting and using a component or an additive used in the formulation of medicaments, quasi-drugs, cosmetics and the like, if necessary, without impairing the effect of the present invention in addition to the aforementioned essential component. The antiallergic agent of the present invention can be used as single medicaments, and also can be contained and used in medicaments, quasi-drugs, cosmetics for skin and hair and the like.

The dosage form of the antiallergic agent of the present invention is not particularly limited, and any form suitable for the purpose can be selected from dosage forms for oral or parenteral administration.

Examples of dosage forms suitable for oral administration include a tablet, a capsule, a powder, a drink, a granule, a parvule, a syrup, a solution, an emulsion, a suspension, a chewable tablet, and the like. Examples of dosage forms suitable for parenteral administration include, but are not limited to, an injection (e.g. a subcutaneous, intramuscular or intravenous injection, and the like), an external preparation, a drop, an inhalant, an air spray, dose drops, eye drops.

Liquid formulations suitable for oral administration such as a solution, emulsion or syrup can be produced using water; sugars such as sucrose, sorbit or fructose; glycols such as polyethylene glycol or propylene glycol; oils such as sesame oil, olive oil or soybean oil; antiseptics such as p-hydroxybenzoate; and flavors such as strawberry flavor and peppermint. In order to produce solid formulations such as capsule, tablet, powder or granule, there can be used an excipient such as lactose, glucose, sucrose or mannite; a disintegrator such as starch or sodium alginate; a lubricant such as magnesium stearate or talc; a binder such as polyvinyl alcohol, hydroxypropylcellulose or gelatin; a surfactant such as fatty acid ester; and a plasticizer such as glycerine, and the like.

Formulations for an injection or drop that is suitable for parenteral administration preferably comprise, as an active ingredient, the above substance in a dissolved or suspended state in a sterilized aqueous medium which is isotonic to the recipient's blood. For example, in the case of an injection, a solution can be prepared using an aqueous medium consisting of a saline solution, a glucose solution or a mixture of a saline solution and a glucose solution. In the case of a formulation for intestinal administration, it can be prepared using carriers such as theobroma oil, hydrogenated lipids or hydrogenated carboxylic acid, and can be provided as a suppository. In order to produce an air spray, the above substance as an active ingredient may be dispersed as microparticles, and a carrier which does not irritate the recipient's cavitas oris and respiratory tract mucosa and which facilitates absorption of the active ingredient can be used. Specific examples of carriers include lactose, glycerine, and the like. Formulations having a form such as aerosol or dry powder may be prepared depending on the properties of the substance of an active ingredient and the carrier to be used. One or two or more auxiliary ingredients selected from glycols, oils, flavors, an antiseptic, an excipient, a disintegrator, a lubricant, a binder, a surfactant, a plasticizer and the like may be added to these formulations for parenteral administration.

In the case where the antiallergic agent of the present invention is used for therapy or prevention of allergic rhinitis, it can be used in the form of nose drops. In addition to the aforementioned mixture of poly lactic acid as an active ingredient, the nose drops of the present invention may be added with a substance which is commonly used in nose drops, such as anti-inflammatory agents (dipotassium glycyrrhizinate, methyl salicylate, acetaminophen, acetyl salicylate, salicylic glycol, indomethacin, and the like), topical anesthetics (lidocaine, lidocaine hydrochloride, dibucaine, dibucaine hydrochloride, benzocaine, ethyl aminobenzoate, and the like), bactericides (acrinol, cetylpyridinium chloride, benzethonium chloride, benzalkonium chloride, chlorhexidine hydrochloride, chlorhexidine gluconate, and the like), vitamins (vitamin A, vitamin C, vitamin B₁₂, and the like), refrigerants (menthol, camphor, eucalyptus oil and the like), thickening agents (gelatin, polyacrylic acid, sodium polyacrylate, polyvinyl alcohol, polyvinyl pyrrolidone, polyethylene oxide, carboxymethyl cellulose, hydroxypropylcellulose, polyvinyl carboxy copolymer, polyvinyl pyrrolidone vinyl acetate copolymer, methylvinylether maleic anhydride copolymer, natural rubber and the like), stabilizers and the like, so long as the effect of the present invention is not impaired.

The dose and dosage frequency of the antiallergic agent of the present invention are determined as appropriate, depending on various factors such as purpose of administration, dosage form, condition such as age, body weight or sex of a patient. Generally, the dose of an active ingredient per day is 1 to 10,000 mg/kg, preferably 10 to 2000 m/kg, and more preferably 10 to 200 mg/kg. It is preferred that the above dose of the agent is dividedly applied about once to 4 times per day.

The time of administration of the antiallergic agent of the present invention is not particularly limited, and the agent can be administered before or after an antigen comes in the body, or before, during or after the antigen-antibody reaction (allergic reaction) by the antigen which has come in the body, or the agent can be administered continuously over two or more of the aforementioned periods.

The present invention also relates to antiallergic food and drink which comprises a mixture of cyclic and/or straight chain poly lactic acids having a condensation degree of 3 to 20. Thus, the mixture of cyclic and/or straight chain poly lactic acids having a condensation degree of 3 to 20 which is used in the present invention is used not only as a form of single agent as mentioned above, but also may be mixed in food and drink, and used.

The preparation form of the antiallergic food and drink of the present invention is not particularly limited, so long as a mixture of poly lactic acid can be contained without being decomposed.

Specific examples of products of the antiallergic food and drink according to the present invention include health foods or supplements including drinks, such as those generally called a soft drink, drinkable preparation, health food, specified supplement food, functional food, function-activating food, nutritional supplementary food, supplement, feed, feed additive and the like.

Specific examples of food and drink include confectionary, such as a chewing gum, chocolate, candy, sweet tablet, jelly, cookie, biscuit and yogurt; frozen deserts, such as ice cream and sherbet; beverages, such as tea, soft drink (including juice, coffee, cocoa and the like), nutrition supplement drinkable preparation, and cosmetic drinkable preparation; and all other food and drink, such as bread, ham, soup, jam, spaghetti, and frozen food. Alternatively, the mixture of poly lactic acids used in the present invention can also be used by adding to seasoning, food additives, and the like. By the use of the antiallergic food and drink of the present invention, there can be provided safe food and drink which can exert antiallergic effect and show substantially no toxic side effect.

The antiallergic food and drink of the present invention can be obtained by directly mixing and dispersing a mixture of poly lactic acids in a common raw material used in food, and then processing the mixture into a desired form by a know method.

The antiallergic food and drink of the present invention encompasses food and drink in every form, and the types are not specifically limited. That is, the food and drink can be provided by mixing the antiallergic agent of the present invention into the above-mentioned various food and drink, or various nutrient compositions, such as various oral or enteral nutrient preparations or drinks. Compositions of such food and drink may include protein, lipid, carbohydrate, vitamin and/or mineral, in addition to the mixture of cyclic and/or straight chain poly lactic acids having a condensation degree of 3 to 20. The form of the food and drink is not specifically limited, and may be in any form, such as solid, powdery, liquid, gel, and slurry forms, so far as it is in a form that is easily ingested.

The content of the mixture of poly lactic acids in the food and drink is not specifically limited, and is generally 0.1 to 20 weight %, more preferably approximately 0.1 to 10 weight %.

The mixture of poly lactic acids is preferably contained in the food and drink in an amount which achieve an antiallergic effect which is an object of the present invention. Preferably, about 0.1 g to 10 g, more preferably about 0.5 g to 3 g, of the mixture of poly lactic acids is contained per food or drink to be ingested.

The content of the specification of Japanese Patent Application No.2001-4823 which the present application claims a priority based on is incorporated herein by reference as a part of the disclosure of the present specification.

The present invention is further described in the following examples, but the scope of the present invention is not limited by the examples in any way.

### EXAMPLES

### Production Example 1: Production of a mixture of poly lactic acids (hereinafter referred to as CPL)

500 ml of L-lactic acid (to which D-lactic acid was also mixed) was placed into a separable flask in a mantle heater. 300ml/min of nitrogen gas was flowed therein while stirring. Accumulated water was introduced into a flask equipped with a reflux condenser via a warmed descending type connecting tube, while heating at 145°C for 3 hours. Furthermore, after pressure was reduced to 150 mmHg and heated at the same temperature for 3 hours, the mixture was heated at 155°C for 3 hours under a reduced pressure of 3 mmHg, and then at 185°C for 1.5 hours under a reduced pressure of 3 mmHg to obtain poly lactic acids as a reaction product.

The obtained poly lactic acids were kept at 100°C, and 100ml of ethanol and 400ml of methanol were separately added thereto, and then the mixture was allowed to be cooled. This mixture was added to 500ml of methanol, and the mixture was well stirred and left to stand. Then, the mixture was filtrated for purification. The filtrate was subjected to vacuum drying and then dissolved in acetonitrile to obtain 200ml (stock solution) in total.

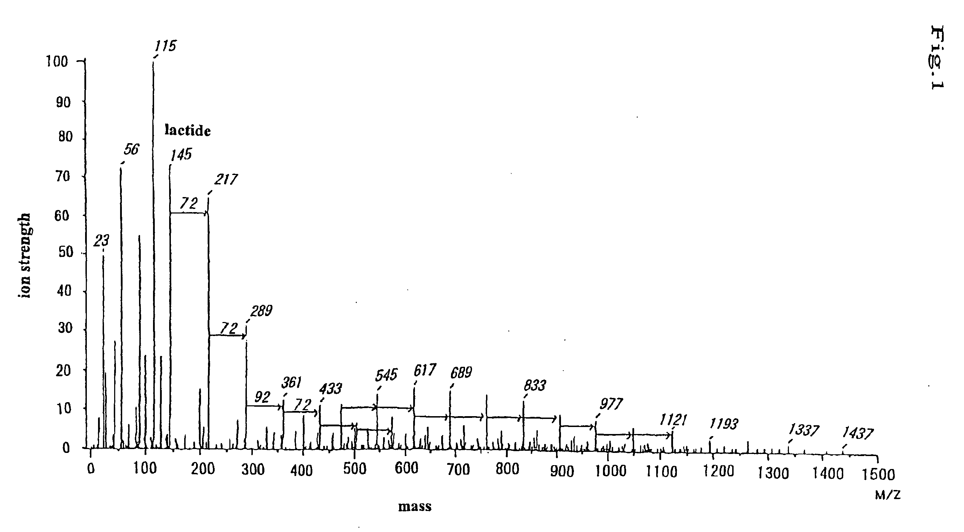

The stock solution was subjected to a reverse phase ODS column (TSK gel ODS-80 TM) which was previously equilibrated, and was stepwise eluted with 30%, 50% and 100% acetonitrile (pH2.0) each containing 0.01M hydrochloric acid to obtain poly lactic acids (condensation degree of 3 to 20) as an acetonitrile 100% elution fraction. The mass spectrum of the obtained substance is shown in Fig. 1. As is clear from the regular fragment ion peaks in Fig. 1, the obtained mixture of poly lactic acids mainly comprises cyclic condensate, and a small amount of linear condensate is contained therein.

### Test Example 1:

### (Test method)

### 1. Test animal

4-week old Hartley-line male Guinea pigs (Japan SLC) were used.

### 2. Drug

The drug used was the mixture of polylactic acids (CPL) prepared in Production Example 1. CPL was dissolved in glycerin at 100 mg/ml, and then purified water was added to make 100 mg/2ml.

### 3. Antigen and adjuvant

For sensitization, the cedar pollen extract antigen prepared by previous papers (Takeshi Nabe et al. (1997) Jpn J. Pharmacol. 75: 243-251 and Takeshi Nabe et al. (1998) Inflamm. Res. 47: 369-374) was used. To cause the reaction by antigen inhalation, cedar pollens were used as they are. The adjuvant used was aluminum hydroxide gel Al(OH)₃ which was prepared by previous papers (Takeshi Nabe et al. (1997) Allergol. Intl. 46: 261-267).

The cedar pollen extract antigen was adsorbed to Al(OH)₃ in accordance with previous papers (Takeshi Nabe et al. (1997) Jpn J. Pharmacol. 75: 243-251 and Takeshi Nabe et al. (1998) Inflamm. Res. 47: 369-374) by dropping an equal amount of aqueous saline solution of the cedar pollen extract antigen (200 µ g protein/ml) on a saline suspension of Al(OH)₃ (200 mg/ml) under stirring.

### 4. Sensitization and reaction occurrence

The sensitization and the reaction occurrence by antigen inhalation were carried out by the method shown in Fig. 2. The cedar pollen extract antigen adsorbed onto Al(OH)₃ was applied to both nasal cavities of the Guinea pig twice a day for 7 days continually to cause sensitization. Thereafter, once a week, using a quantitative pollen inhaler (described in Takeshi Nabe et al. (1997) Jpn J. Pharmacol. 75: 243-251), cedar pollen particles were repeatedly inhaled by the Guinea pig under spontaneous respiration to cause the reaction occurrence up to 23 times.

### 5. Measurement of sneeze

Frequencies of sneeze were counted by observing symptoms of the Guinea pig immediately after cedar pollen inhalation to 1 hour since then. Almost no expression of sneeze is observed after 1 hour and later as reported in the previous paper (Takeshi Nabe et al. (1998) Inflamm. Res. 47: 369-374).

### 6. Measurement of respiration function

Specific airway resistance (sRaw), that was used as an index of a nasal resistance after the reaction occurred by inhalation of cedar pollens, was measured by using a multifunction respiration meter (Pulmos-I, M.I.P.S) using two chambered, double flow plethysmograph method.

### 7. Measurement of reactivity to histamine application to a nose

Reactivity to histamine application to a nose was measured in accordance with the previous paper (Nobuaki, Mizutani et al. Eur. Respir. J. 14: 1368-1375 (1999)). That is, 2 days after the 23rd reaction occurrence, 10 µ l/nostril (20 µ l/animal of 10⁻⁴ and 10⁻² M histamine solution was applied to both the cavities orderly in 20 minutes intervals. sRaw as the index of the reaction was measured 10 minutes after each nasal application.

### 8. Administration of drugs (Fig. 2)

CPL (100 mg/2 ml/kg) was orally administered for consecutive days, once a day, starting at 6th day before the 23rd reaction occurrence. The final administration was carried out at 2 hours before the 23rd reaction occurrence. To a control group was orally administered a 50% glycerin solution in the same manner.

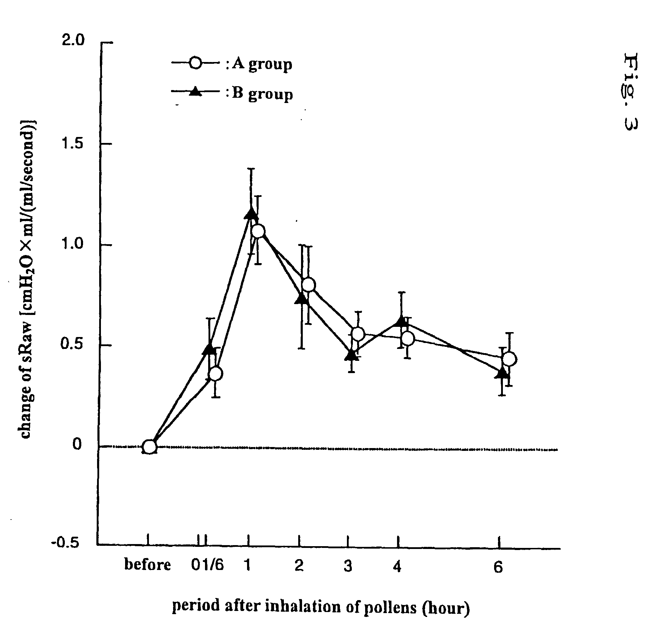

The control group and a CPL administration group were divided into two groups to make the reaction degree same by observation of a degree of increase of sRaw after the 22nd reaction occurrence (Table 1 and Fig. 3). In Fig. 3, each point shows mean ±S. E. of 15 or 16 model animals.

**Table 1:**

| Area under a reaction curve (AUC) of an increase of specific airway resistance (sRaw) in the early period (0-3 hours) and the later period (3-6 hours) after the 22nd antigen administration in sensitized Guinea pig. | | | |
|---|---|---|---|
| | | Increase of sRaw | |
| | | Early period | Later period |
| Group | Number of animal | [AUC(0 to 3 hour)] | [AUC(3 to 6 hour)] |
| A | 16 | 2.27±0.36 | 2.01±0.43 |
| B | 15 | 2.30±0.46 | 1.99±0.46 |
| Each value shows mean ±S. E. of 15 or 16 model animals. | | | |

### 9. Statistical analysis.

For a statistical analysis was used Bonferroni's multiple test with a level of significance <5% as being significant.

### (Result of evaluation)

### 1. Effect of CPL on an increase of two-phase nasal cavity resistance

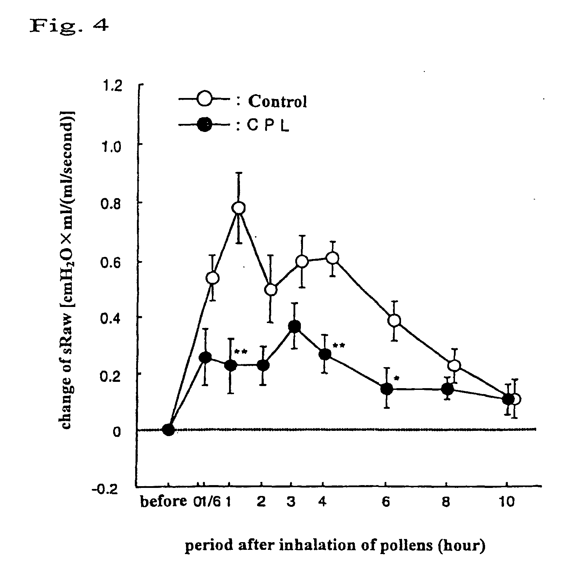

Fig. 4 shows scores of an effect of CPL on an increase of sRaw which is an index of the nasal cavity resistance after the reaction occurrence. In Fig. 4, CPL (100 mg/kg/dose/day) was orally and continuously administered for 7 days. The final administration was carried out at 2 hours before the 23rd antigen inhalation administration. Each dot represents mean ± S. E. of 15 or 16 model animals. For significant differences from the control group, a single asterisk represents p<0.05 and double asterisks represents p<0.01.

As is understood from Fig. 4, the control group showed an instant nasal obstruction showing the increase of sRaw at 1 hour after the reaction occurrence, and a delayed reaction showing the second increase of sRaw after 3 to 4 hours. In contrast with this two-phase nasal obstruction, CPL showed suppression or its tendency over a whole time zone against both of the instant and the delayed reactions.

Table 2 shows the scores of Fig. 3 represented by scores of the area under a reaction curve (AUC) of the increase of individual nasal cavity resistances immediately to 3 hours after and at 3 to 10 hours after the reaction occurrence. Against the nasal obstruction of the immediate phase and the delayed phase, CPL showed significant (p<0.01) suppression of about 60% and 50%, respectively.

**Table 2:**

| Effect of CPL on an increase of the area under a reaction curve (AUC) of specific airway resistance (sRaw) in the early period (0-3 hours) and the later period (3-10 hours) after the 23rd antigen inhalation administration in sensitized Guinea pig | | | |
|---|---|---|---|
| | | Increase of sRaw | |
| | | Early period | Later period |
| Group | Number of animal | [AUC(0 to 3 hour)] | [AUC(3 to 10 hour)] |
| Control | 16 | 1.79±0.23 | 2.55±0.27 |
| CPL | 15 | 0.76±0.19** | 1.31±0.29** |

CPL (100 mg/kg/dose/day) was orally and continuously administered for 7 days. The final administration was carried out at 2 hours before the 23rd antigen inhalation administration. Each value represents mean ± S. E. of 15 or 16 model animals. For significant differences from the control group, double asterisks represents p<0.01.

### 2. Effect of CPL on the expression of sneeze

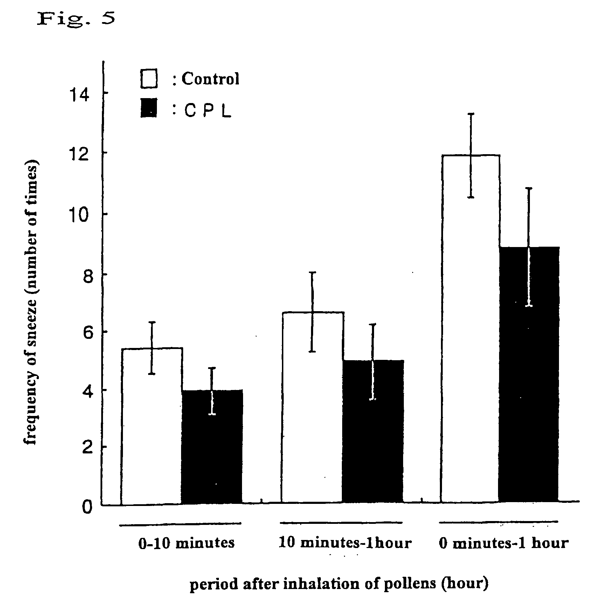

Fig. 5 shows scores of the effect of CPL on the expression of sneeze immediately and at 1 hour after the reaction occurrence in the sensitized Guinea pig. In Fig. 5, CPL (100 mg/kg/dose/day) was orally and continuously administered for 7 days. The final administration was carried out at 2 hours before the 23rd antigen inhalation administration. Each bar represents mean ±S.E. of 15 or 16 model animals.

As is understood from Fig. 5, the control group showed about 6 times of sneeze expression in each of 0 to 10 minutes and 10 minutes to 1 hour after the reaction occurrence. On the contrary, CPL administered group merely showed a small suppressing tendency.

### 3. Effect of CPL on the expression of nasal anaphylaxis against histamine

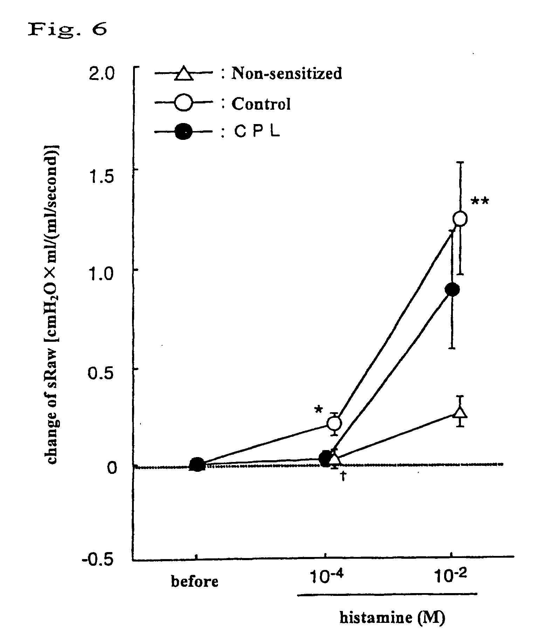

Fig. 6 shows scores of the effect of CPL on the expression of nasal anaphylaxis against histamine. The experiment was carried out at 2 days after 23rd administration by pollen inhalation. CPL (100 mg/kg/dose/day) was orally and continuously administered for 7 days. The final administration was conducted at 2 hours before the 23rd antigen inhalation administration. Each dot represents mean ± S. E. of 11 to 16 model animals. For the significant difference from the non-sensitized group, the single asterisk represents p<0.05 and the double asterisks represents p<0.01, and a cross denotes that the significant difference from the control group is p<0.05.

As is understood from Fig. 6, the non-sensitized Guinea pig merely showed a small increase of sRaw in 10⁻² M of histamine used for nasal application. Sensitization-caused Guinea pigs showed the concentration-dependent increase of sRaw starting from 10⁻⁴ M. To this sthenia of nasal anaphylaxis, CPL suppressed significantly (p<0.05) the reaction in 10⁻⁴ M and also showed the suppressing tendency in the reaction in 10⁻² M.

### (Summary of Test Examples)

Effects of CPL on the sneeze, the two-phase nasal obstruction and the expression of nasal anaphylaxis to the nasal application of histamine in the allergic rhinitis model of the Guinea pig, were examined. Continuous administration of CPL (100 mg/2 ml/kg, po) for 7 days did not show clear suppression to the expression of sneeze, but clearly suppressed both the instant and delayed two-phase nasal obstructions, and also suppressed the expression of nasal anaphylaxis to histamine.

### Industrial Applicability

The antiallergic agent of the invention can be used for therapy and prevention of allergic diseases such as allergic rhinitis.

The mixture of poly lactic acids used in the present invention as an active ingredient is a low condensate of lactic acids derived from organism components, and therefore shows a high biocomparability and no side effects which are observed in the case of antihistamic agents used conventionally for therapy of allergic diseases, antiallergic agents having an action of suppressing release of chemical mediator from mast cells, or steroid hormones.

## Claims

1. An antiallergic agent which comprises a mixture of cyclic and/or straight chain poly lactic acids having a condensation degree of 3 to 20.

2. The antiallergic agent of claim 1 which is used as a therapeutic agent or a preventive agent of allergic diseases.

3. The antiallergic agent of claim 2 wherein the allergic diseases is the type I allergy disease.

4. The antiallergic agent of claim 2 or 3 wherein the allergic diseases is allergic rhinitis.

5. The antiallergic agent of any one of claims 1 to 4, wherein the lactic acid that is a repeating unit in the poly lactic acid consists substantially of L-lactic acid.

6. The antiallergic agent of any one of claims 1 to 5, wherein the mixture of cyclic and/or straight chain poly lactic acids having a condensation degree of 3 to 20 is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

7. The antiallergic agent of claim 6, wherein condensation by dehydration is performed by stepwise decompression and temperature rise under nitrogen gas atmosphere.

8. The agent of claim 6 or 7, wherein reverse phase column chromatography is performed by ODS column chromatography.

9. Antiallergic food and drink, which comprises the antiallergic agent of any one of claims 1 to 8.
